(19) Europäisches Patentamt
European Patent Office
Office européen des brevets

(11) Veröffentlichungsnummer: **0 042 092**
**A2**

(12) # EUROPÄISCHE PATENTANMELDUNG

(21) Anmeldenummer: **81104162.3**

(22) Anmeldetag: **01.06.81**

(51) Int. Cl.³: **C 07 D 211/90**, C 07 D 401/12, A 61 K 31/435

(30) Priorität: **12.06.80 DE 3022002**

(43) Veröffentlichungstag der Anmeldung: **23.12.81 Patentblatt 81/51**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Materne, Carsten, Dr., Hans Böckler-Strasse 31, D-5300 Bonn 3 (DE)**

(54) **1,4-Dihydropyridin-carbonsäureamide Verfahren zu deren Herstellung, ihre Verwendung in Arzneimitteln sowie deren Herstellung.**

(57) Die Erfindung betrifft neue 1,4-Dihydropyridin-4-carbonsäureamide der allgemeinen Formel I

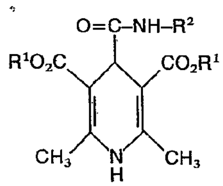

I

in welcher $R^1$ und $R^2$ die in der Beschreibung angegebene Bedeutung haben, ein Verfahren zu ihrer Herstellung, ihre Verwendung in Arzneimitteln, insbesondere in Arzneimitteln mit kreislaufbeeinflussender Wirkung sowie deren Herstellung.

EP 0 042 092 A2

0042092

BAYER AKTIENGESELLSCHAFT 5090 Leverkusen, Bayerwerk

Zentralbereich
Patente, Marken und Lizenzen KS/Kü-c

# 1,4-Dihydropyridin-4-carbonsäureamide, Verfahren zu deren Herstellung, ihre Verwendung in Arzneimitteln, sowie deren Herstellung

Es ist bereits bekannt, daß 1,4-Dihydropyridinderivate Kreislauf-beeinflussende Eigenschaften aufweisen. So ist z.B. der 2,6-Dimethyl-4-(2'-nitrophenyl)-1,4-dihydropyridin-3,5-dicarbonsäuredimethylester (Nifedipin, vergl. DT-PS 1 607 827) als Verbindung bekannt, die coronarvasodilatierende Wirkung besitzt.

1,4-Dihydropyridin-4-carbonsäureamide sind bisher nicht beschrieben. Die Erfindung betrifft daher neue 1,4-Dihydropyridine der Formel I

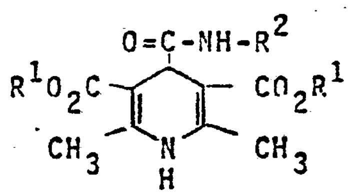

I

in der $R^1$ einen geradkettigen oder verzweigten Kohlenwasserstoff von 1 - 4 C-Atomen bedeutet und $R^2$ für einen verzweigten oder geradkettigen Alkylrest von 1 - 4 C-Atomen oder für

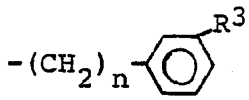 Rest, bei dem n = 0,1,2 und $R^3$ ein oder zwei Methyl-, Hydroxy- oder Methoxygruppen bedeuten, oder für einen

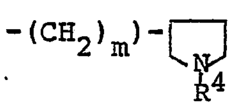 Rest, bei dem m = 1,2 und $R^4$ eine niedere Alkylgruppe bedeutet, oder für einen $-(CH_2)_n-$

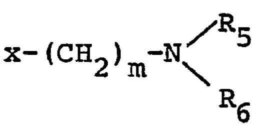 (m = 1,2) Rest, wobei $R^5$, $R^6$ eine niedere Alkylgruppe uns x eine $CH_2$-Gruppe oder ein Heteroatom wie O oder S bedeuten oder für einen

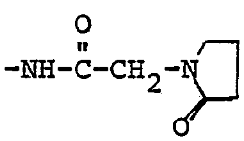 Rest steht.

Die neuen Verbindungen besitzen Kreislauf-beeinflussende Eigenschaften, insbesondere erweitern sie die Coronargefäße und senken den Blutdruck.

Die Herstellung der neuen Verbindungen kann dadurch erfolgen, daß man 1,4-Dihydropyridine der Formel II

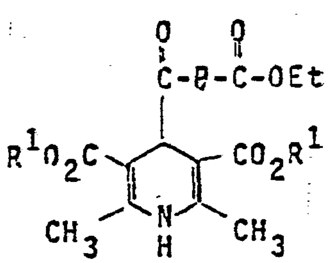 II

in der $R^1$ die oben angegebene Bedeutung besitzt, mit Aminen der Formel III

$$H_2N-R^2 \qquad III$$

in der $R^2$ die oben angegebene Bedeutung beseitzt, in einem inerten organischen Lösungsmittel umsetzt.

Als organische Lösungsmittel können Alkohole, Dioxan, Essigester, Aceton, Chloroform oder Mischungn davon sowie Mischungen mit Wasser genommen werden. Die Reaktionstemperaturen können in einem Bereich zwischen 0°C und 100°C variiert werden. Vorzugsweise arbeitet man bei Raumtemperatur.

Die verwendbaren Ausgangsstoffe der Formel II sind bekannte Verbindungen oder können nach bekannten Verfahren hergestellt werden.

Als Amine der Formel III können beispielsweise eingesetzt werden: 3,4-Dimethoxyphenylethylamin, 3,4-Dihydroxyphenylethylamin, 3,4-Dimethoxybenzylamin, Propylamin, Isopropylamin, sec. Butylamin, Benzylamin, 3,4-Dimethoxybenzylamin, 1-Ethyl-2-aminomethylpyrrolidin, Diethylaminoethylmercaptoethylamin, Dimethylaminoethoxyethylamin, 1-Pyrrolidin-2-(on)-essigsäurehydrazid.

Die neuen Verbindungen sind als Arzneimittel verwendbare Substanzen. Sie haben ein breites und vielseitiges pharmakologisches Wirkungsspektrum.

Im einzelnen konnten im Tierexperiment folgende Hauptwirkungen nachgewiesen werden:

1) Die Verbindungen bewirken bei parenteraler, oraler und perligualer Zugabe eine deutliche und langanhaltende Erweiterung der Coronargefäße. Diese Wirkung auf die Coronargefäße wird durch einen gleichzeitigen Nitritähnlichen herzentlastenden Effekt verstärkt.

Sie beeinflussen bzw. verändern den Herzstoffwechsel im Sinne einer Energieersparnis.

2) Die Erregbarkeit des Reizbildungs- und Erregungsleistungssystems innerhalb des Herzens wird herabgesetzt, so daß eine in therapeutischen Dosen nachweisbare Antiflimmerwirkung resultiert.

3) Der Tonus der glatten Muskulatur der Gefäße wird unter der Wirkung der Verbindungen stark vermindert. Diese gefaßspasmolytische Wirkung kann im gesamten Gefäßsystem stattfinden, oder sich mehr oder weniger isoliert in umschriebenen Gefäßgebieten (wie z.B. dem Zentralnervensystem manifestieren.

4) Die Verbindungen senken den Blutdruck von normotonen und hypertonen Tieren und können somit antihypertensive Mittel verwendet werden.

5) Die Verbindungen haben stark muskulär-spasmolytische Wirkungen, dies an der glatten Muskulatur des Magens, Darmtraktes, des Urogenitaltrakes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gewichtsprozent der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser nichttoxische organische Lösungsmittel, wie Paraffine (z.B. Erdölfraktionen), pflanzliche Öle (z.B. Erdnuß-/Sesamöl), Alkohole (z.B. Ethylalkohol,

Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene anionische Emulgatoren (z.B. Polyoxyethylen-Fettsäure-Ester), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perligual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Im allgemeinen hat es sich als vorteilhaft erwiesen, bei intravenöser Applikation Mengen von etwa 0,01 bis 10 mg/kg vorzugsweise etwa 0,1 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch auf Grund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Im folgenden sei die Erfindung anhand einiger Beispiele zur Herstellung der neuen Dihydropyridine erläutert.

Beispiel 1

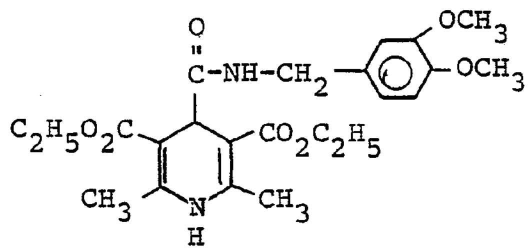

2,6-Dimethyl-1,4-dihydropyridin-3,5-tricarbonsäure-3,5-diethylester-4-(3'4'-dimethoxybenzyl)-amid.

34,5 g des gemischten Anhydrids aus 2,6-Dimethyl-1,4-dihydropyricin-3,4,5-tricarbonsäure-3,5-diethylester und Kohlensäureethylester werden in 50 $cm^3$ Aceton mit 15,7 g 3,4-Dimethoxybenzylamin bei Raumtemperatur versetzt. Man läßt 2 Stunden rühren, engt ein und kristallisiert den Rückstand aus Ethanol um.
Ausbeute: 31,2 g (70 %)
Schmelzpunkt: 186 - 190°C.

In analoger Weise erhält man:

2,6-Dimethyl-1,4-dihydropyridin-3,4,5-tricarbonsäure-3,5-diethylester-4-isopropylamid

2,6-Dimethyl-1,4-dihydropyridin-3,4,5-tricarbonsäure-3,5-dimethylester-4-butylamid

2,6-Dimethyl-1,4-dihydropyridin-3,4,5-tricarbonsäure-3,5-diethylester-4-/2-(3,4-dihydroxyphenyl)-ethyl/-amid

2,6-Dimethyl-1,4-dihydropyridin-3,4,5-tricarbonsäure-3,5-diethylester-4-(3-diethylaminopropyl)-amid

2,6-Dimethyl-1,4-dihydropyridin-3,4,5-tricarbonsäure-3,5-diethylester-4-/2-(2-diethylaminoethylmercapto)-ethyl/-amid

2,6-Dimethyl-1,4-dihydropyridin-3,4,5-tricarbonsäure-3,5-diethylester-4-(2,6-dimethyl)-anilid

2,6-Dimethyl-1,4-dihydropyridin-3,4,5-tricarbonsäure-3,5-diethylester-4-/(1-ethylpyrrolidinyl)-methyl/-amid

Beispiel 2

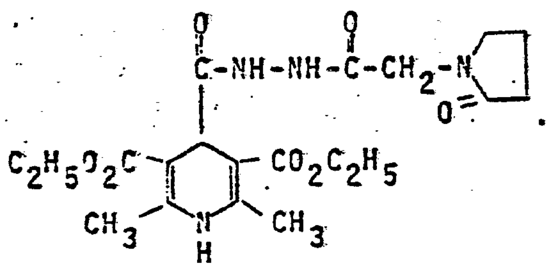

2,6-Dimethyl-1,4-dihydropyridin-3,4,5-tricarbonsäure-3,5-diethylester 4-N'-(1-Pyrrolidinon(2)yl)acetyl-hydrazid.

34,5 g des gemischten Anhydrids aus 2,6-Dimethyl-1,4-dihydropyridin-3,4,5-tricarbonsäure-3,5-diethylester und Kohlensäureethylester in 100 cm³ Chloroform werden bei Raumtemperatur mit 16,3 g 1-Pyrrolidinon-(2)-ylessigsäurehydrazid in 20 cm³ $CHCl_3$ tropfenweise versetzt. Man läßt 1 bis 2 Stunden rühren, saugt ab und kristallisiert aus Methanol/Wasser um.
Ausbeute: 31,8 g (73 %)
Schmelzpunkt: 246 - 247°C.

Patentansprüche

1. 1,4-Dihydropyridine der Formel I

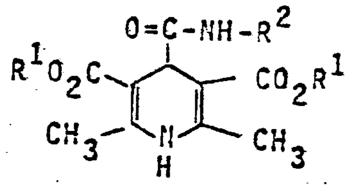

I

in der $R^1$ einen geradkettigen oder verzweigen Kohlenwasserstoff von 1 - 4 C-Atomen bedeutet und $R^2$ für einen verzweigten oder geradkettigen Alkylrest mit 1 - 4 C-Atomen oder für einen

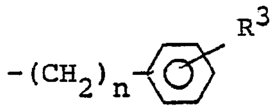 Rest, bei dem n = 0, 1, 2 und $R^3$

ein oder zwei Methyl-, Hydroxy- oder Methoxygruppen bedeuten, oder für einen

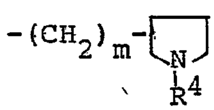 Rest, bei dem m = 1, 2 und $R^4$ eine

niedere Alkylgruppe bedeutet, oder für einen

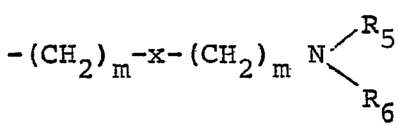 (m = 1,2) Rest, wobei $R^5$,

$R^6$ eine niedere Alkylgruppe und x eine $CH_2$-Gruppe oder ein Heteroatom wie O oder S bedeuten, oder für

einen 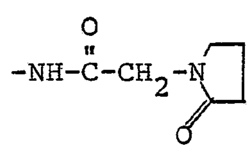 Rest steht.

2. Verfahren zur Herstellung von 1,4-Dihydropyridinen nach Anspruch 1, dadurch gekennzeichnet, daß man 1,4-Dihydropyridine der Formel II

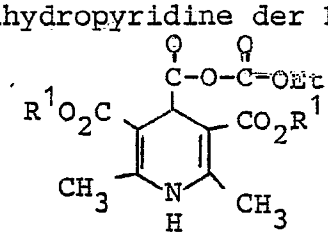

II

in der $R^1$ die in Anspruch 1 angegebene Bedeutung besitzt, mit Aminen der Formel III

$$H_2N-R^2$$ III

in der $R^2$ die in Anspruch 1 angegebene Bedeutung besitzt, in einem inerten organischen Lösungsmittel umsetzt.

3. Arzneimittel, gekennzeichnet durch einen Gehalt an 1,4-Dihydropyridin-Derivaten gemäß Anspruch 1 und üblichen pharmazeutischen Hilfsstoffen.

4. Verfahren zur Herstellung von Arzneimitteln, dadurch gekennzeichnet, daß man 1,4-Dihydropyridin-Derivate gemäß Anspruch 1, gegebenenfalls unter Verwendung von Hilfs- und Trägerstoffen in eine geeignete Applikationsform überführt.

5. Verwendung von 1,4-Dihydropyridin-Derivaten bei der Bekämpfung von Erkrankungen.

6. Verwendung von 1,4-Dihydropyridin-Derivaten in Arzneimitteln zur Bekämpfung von Kreislauferkrankungen.